# EUROPEAN PATENT APPLICATION

(11) **EP 3 992 630 A1**
(43) Date of publication of application: **04.05.2022**
(21) Application number: 20205072.0
(22) Date of filing: 30.10.2020
(51) Int. Cl.: G01N 33/574

(54) **METHODS AND KITS TO MONITOR SQUAMOUS CELL CARCINOMA**

(71) Applicant: Universitätsspital Basel, 4031 Basel (CH); Biovesicle Inc, Taipei 10047 (TW)
(72) Inventor: Muller, Laurent, 4031 Basel (CH); Chiang, Dapi Menglin, 85356 Freising (DE)
(74) Representative: Lang, Christian

(57) **Abstract**

The present invention relates to a method and kit for the diagnosis and prognosis of squamous cell carcinoma or an elevated risk of future occurrence thereof by measuring at least one biomarker correlating with a diagnosis or prognosis of squamous cell carcinoma in a sample obtained from a subject, wherein the correlation takes into account the presence and level of the at least one biomarker in the sample as compared to a reference sample from a healthy subject, wherein the methods and kits of the present invention use at least one exosomal biomarker selected from a group consisting of exosomal Pan-EV; Pan-CK; EpCAM; PD-L1 and CD45. The present invention further relates to a method for screening therapeutic antibodies for selecting the most suitable antibody for use in treating squamous cell carcinoma for an individual patient.

## Description

### TECHNICAL FIELD

The present disclosure relates to methods and kits to monitor cancer, in particular squamous cell carcinoma in samples obtained from a subject having or being at risk of developing squamous cell carcinoma.

### BACKGROUND

Head and neck squamous cell carcinoma (HNSCC) is one of the most frequently observed cancers worldwide. Even though major advances in diagnosis and treatment have been made, the 5-year survival rate is still poor and for patients with an advanced cancer stage, there is a high risk of recurrence within two years.

Current diagnosis and monitoring techniques, such as radiography or endoscopy, require vast medical experience, often lead to delayed results and may expose the patient to a risk of possible side effects. Moreover, solid tissue biopsy samples are commonly needed for diagnosis. In view of the foregoing, there exists a need to provide non-invasive diagnosis and monitoring tools for detecting squamous cell carcinoma, in particular for HNSCC.

According to document US 2018/0171413 A1 tumor DNA in saliva and plasma is a valuable biomarker to detect HNSCC. Document WO 2019/126249 A1 discloses a method to detect proteins associated with HNSCC by measuring the amount of an expression product from at least one specific gene in a sample from an individual. The genes may comprise at least one of CAB39L, ADAM12, SH3BGRL2, NRG2, COL13A1, GRIN2D, LOXL2, KRT4, EMP1 or HSD17B6. When comparing the expression of at least one gene with a control value for the expression product of the gene, a difference between gene expression in the sample and the control value indicates whether the individual has or is at increased risk for developing HNSCC. Further, document WO 2017/077499 A1 also deals with the provision of biomarkers to identify HNSCC and corresponding methods of detecting cancer and determining or predicting recurrence. Disclosed therein is a method of predicting recurrence of malignancy in a subject having or suspected of having HNSCC by detecting aberration in at least one selected gene. Documents WO 2007/133725 A1 and US 2016/0341728 A1 respectively teach another non - invasive method of diagnosing HNSCC in a subject, which uses assaying for the presence of at least one biomarker in a sample and correlating a detection value of the at least one biomarker with occurrence of or an increased risk of developing HNSCC, wherein the correlation takes into account the detection of the one or more biomarker in the sample compared to a frequency or level of the biomarker in a reference sample, wherein the biomarker is selected from hyaluronic acid (HA); hyaluronidase (HAase) and CD44.

However, the underlying analysis procedures are heavily time - consuming and expensive, so that there still exists a strong need to provide better methods and kits to monitor squamous cell carcinoma.

Exosomes are small vesicles (about 30-150 nm) secreted from different cell types and found in various biological fluids, such as blood, urine, saliva and central spinal fluid (CSF). Exosomes contribute to intercellular signalling, antigen presentation, as well as tumor progression by carrying cellular proteins, RNA/DNA, glycans, and/or lipids. It is well known that exosomes are massively produced by cancer cells (tumor-derived exosomes, TEX), which is why in recent years exosomes have moved into spotlight of research as possible biomarkers for cancer diagnosis, disease monitoring and monitoring of chemotherapeutic response.

However, many conventional protocols to isolate exosomes involve the use of bovine serum albumin (BSA) or human serum albumin (HSA), which may interfere with the isolation efficiency of exosomes. Moreover, common techniques to purify exosome are usually challenging due to the issue of lipoprotein contamination and require special equipment(s) and operational expertise. As disclosed by document EP 3 602 055 A1, a new method for effectively isolating exosomes in a sample without jeopardizing the integrity of the exosomes has been recently developed, which uses EXÖBead^{®} being a non-antibody coated magnetic bead (more specifically, a galectin-based glycan recognition particle capable of capturing exosomes in plasma). This offers new opportunities in exosome research.

Additionally, for treating cancer patients, antibody-based therapies became increasingly important in the recent years. However, for antibody therapy, immunohistochemistry (IHC) staining is traditionally applied for predicting therapeutic response, but this approach requires a solid tumor sample for therapeutic antibody screening. Moreover, traditional antibody therapy is accompanied by a high failure rate, namely 20 % response rate and 80% non-response rate. At present, the treating physician therefore cannot easily choose which antibody therapy is the best option for the individual patient. Furthermore, during antibody therapy, the response cannot be easily tracked and monitored. Hence, there further exists a need for providing new methods of screening therapeutic antibodies for cancer treatment and tracking therapeutic (positive) response during the actual treatment.

### SUMMARY

The present disclosure relates to methods and kits for use in the diagnosis and/ or prognosis of squamous cell carcinoma or an elevated risk of future occurrence thereof, which are suitable for effective and reliable cancer diagnosis, disease monitoring and monitoring of chemotherapeutic response. The present disclosure further relates to a method of screening therapeutic antibodies, which shall be used in the treatment of squamous cell carcinoma, wherein depending on an assay response the therapeutic antibody with the best response can be chosen.

The invention is defined in independent claims 1, 12 and 14. The dependent claims define preferred embodiments.

In the first aspect, the present disclosure is directed to an in vitro method for the diagnosis and/ or prognosis of squamous cell carcinoma or an elevated risk of future occurrence thereof, comprising detection of at least one biomarker correlating with a diagnosis of squamous cell carcinoma or an elevated risk of developing the same, the correlation taking into account the presence and level of the at least one biomarker in the sample as compared to a reference value. In more detail, the method is performed by the following steps: (i) obtaining a sample from a subject; (ii) assaying the sample obtained in step (i) for the presence of at least one biomarker and determining a quantitative value of the least one selected biomarker in the test sample; (iii) comparing the quantitative biomarker value obtained in step (ii) with the corresponding reference value, wherein the reference value is a quantitative value of the least one selected biomarker in a reference sample obtained from a healthy subject; and (iv) evaluating the comparison results obtained in step (iii) to determine, whether the subject from which the test sample is obtained is likely to have squamous cell carcinoma or has an elevated risk of developing the same, wherein the method according to the present invention uses at least one exosomal marker as the biomarker, the marker being selected from a group consisting of exosomal Pan-EV; Pan-CK; Ep-CAM; PD-L1 and CD45.

According to the present disclosure, biomarkers Pan-EV; Pan-CK; EpCAM; PD-L1 and CD45 are exosomal proteins or may be isoforms thereof.

In certain embodiments of the method of the present disclosure, the sample may be assayed for the presence and level of a plurality of said biomarkers.

In some embodiments, all the biomarkers of Pan-EV; Pan-CK; EpCAM; PD-L1 and CD45 may be detected in the sample.

Specifically, the presence or increased level of exosomal Pan-EV; Pan-CK; EpCAM; PD-L1 and CD45 as well as their ratio may be used as indicators to determine a tumor stage.

Therefore, in some embodiments of the present invention, the method may comprise assaying the test sample obtained from the subject for exosomal Pan-EV; Pan-CK; EpCAM; PD-L1 and CD45 and comparing the values of exosomal Pan-EV; Pan-CK; EpCAM; PD-L1 and CD45 and a percentage of Pan-EV+/CD45- or Pan-EV+/CD45+ with triple positive of PD-L1, Pan-CK and EpCAM biomarkers in the test sample with those from a reference sample, wherein elevated values for the exosomal Pan-EV; Pan-CK; EpCAM; PD-L1 and CD45 and the percentage of triple positive Pan-EV+/CD45- or Pan-EV+/CD45+ in the test sample relative to the reference sample correlate with presence of squamous cell carcinoma or an elevated risk of future occurrence thereof.

The subject sample obtained in step (i) may be selected from a group consisting of saliva, blood, blood plasma, serum, urine or other body fluids. In a preferred embodiment, the subject sample may be blood plasma.

According to some embodiments of the present invention, step (ii) may be performed by using multiple antibody plotting.

In a preferred embodiment, EXObead^{®}-based antibodies stained flow cytometry may be applied for detection of the at least one exosomal biomarker in the sample.

Preferably, the sample obtained in step (i) may be from a head and neck cancer patient or subject suspected to have or being at risk of developing head and neck squamous cell carcinoma (HNSCC).

In another aspect, the present disclosure is directed to a screening kit for diagnosis and / or prognosis of squamous cell carcinoma or an elevated risk of future occurrence thereof, the kit comprising a reference sample for at least one biomarker selected from the group consisting of exosomal Pan-EV; Pan-CK; EpCAM; PD-L1 and CD45 and means for detecting presence and level of the at least one biomarker.

According to some embodiments of the present disclosure, the kit may comprise reference samples for all the exosomal biomarkers Pan-EV; Pan-CK; EpCAM; PD-L1 and CD45 and means for detecting and measuring these biomarkers and for determining a percentage of Pan-EV+/CD45- or Pan-EV+/CD45+ with triple positive of PD-L1, Pan-CK and EpCAM.

Another aspect of the present disclosure is directed to a screening method to select the most effective therapeutic antibody / antibodies for use in treating squamous cell carcinoma, comprising provision of a liquid biopsy sample (such as whole blood) from the patient. The method comprises incubating peripheral blood mononuclear cells (PBMCs) isolated from the whole blood sample and analyzing presence of markers CTLA4 and CD69 before and after T - cell stimulation. The peripheral blood mononuclear cells (PBMCs) and / or platelet - poor plasma of the liquid biopsy sample are further treated with an agent containing exosomes and an agent containing pre-selected antibodies. The platelet - poor plasma of the liquid biopsy sample is further analysed for the presence and amounts of exosomal markers Pan-EV, Pan-CK, EpCAM, PD-L1 and CD45, wherein, on the basis of the results, it is determined whether the conditions for therapeutic antibody positive response are met.

According to a preferred embodiment of the present invention, the conditions for therapeutic antibody positive response may only be met, if the following four prerequisites are all fulfilled: (1) the total exosome including tumor - derived exosome suppresses proliferation of the subject's own PBMCs, (2) a percentage of CTLA4-CD69+ is increased after stimulation, (3) a percentage of CTLA4+ CD69- is decreased after stimulation and (4) a percentage of CD45- EV with PanCK+, EpCAM+ and PD-L1 + for the patient's sample is higher than in a comparison sample obtained from healthy subjects.

According to some embodiments of the present disclosure, the exosomes in the exosome-containing agent may be either exosomes obtained from the same subject's sample or may be exosomes obtained from a sample from another subject. Preferably, the exosomes may comprise the total exosome including tumor - derived exosomes.

According to the present disclosure, the pre-selected antibody in the antibody-containing agent may be selected from a group consisting of Durvalumab (PD-L1 inhibitor, Imfinzi^{®}), Cetuximab (EGFR inhibitor, Erbitux^{®}) and Nivolumab (PD1 inhibitor, Opdivo^{®}), but is not limited thereto. It may also include similar antibodies from other clones.

### BRIEF DESCRIPTION OF DRAWINGS

The features and advantages of the present disclosure will become better understood with reference to the following detailed description considered in connection with the accompanying drawings, where:
- FIG. 1: is a work flow diagram illustrating a method for detecting biomarkers on exosomes by using EXÖBead^{®};
- FIG. 2a: is a diagram representing triplicate results of measured particles in plasma by ZetaViewer from one donor; mean size was around 100 nm;
- FIG. 2b: is an image of eluted exosome with EXÖBead^{®} in transmission electron microscopy (TEM);
- FIG. 2c: is an image of eluted exosome with EXÖBead^{®} in cryogenic electron microscopy (cryo-EM);
- FIG. 3: is a schematic diagram showing MFI (mean fluorescence intensity) of exosome-EXÖBead^{®} complex from healthy donors and patients;
- FIG. 4a: is a schematic diagram showing control of exosomal intracellular marker, TSG101, measured following MISEV guidelines;
- FIG. 4b: is a schematic diagram showing control of non-EVs marker, ApoA1, measured following MISEV guidelines;
- FIG. 5a: is a schematic diagram showing MFI of exosome-EXÖBead^{®} complex from healthy donors and patients;
- Figure 5b: is a schematic diagram showing percentage of EV+/ CD45- or EV+/CD45+ with triple positive HNSCC biomarkers PD-L1, Pan-CK and EpCAM;
- Figure 6: is a schematic diagram showing a functional assay of T-cell activation (conventional assay (UC));
- Figure 7: is a schematic diagram showing a functional assay for antibody screening of a HNSCC patient (sample 61) for treatment with an agent containing exosomes from sample 61 or 64, wherein positive therapeutic response is determined;
- Figure 8: is a schematic diagram showing a functional assay for antibody screening of a HNSCC patient (sample 60), wherein no therapeutic response has been observed; and
- Figure 9: is a schematic diagram showing that percentage of EV CD45- with triple positive HNSCC biomarkers PD-L1, Pan-CK and EpCAM must be higher in cancer patients than in healthy donors.

### DETAILED DESCRIPTION

The detailed description provided below in connection with the appended drawings is intended as a description of some illustrative examples of the method and kit according to the present invention and is not intended to represent the only forms in which the present invention may be constructed or utilized. The same or equivalent functions may be also accomplished in another way. Therefore, the sole purpose of the following description is to present some concepts disclosed herein in a simplified form without limiting the scope of the claims to the examples disclosed herein.

For convenience, certain terms employed in the specification, examples and appended claims are collected here. Unless otherwise defined herein, scientific and technical terminologies employed in the present disclosure shall have the meanings that are commonly understood and used by one of ordinary skill in the art.

Unless otherwise required by context, it will be understood that singular terms shall include plural forms of the same and plural terms shall include the singular. Also, as used herein and in the claims, the term "at least one" has the same meaning and includes one, two, three, or more. Furthermore, the phrase "at least one of A, B, and C", as used throughout this specification and the appended claims, is intended to cover A alone, B alone, C alone, A and B together, B and C together, A and C together, as well as A, B and C together.

As used herein, the term "exosome" refers to extracellular vesicles released from cells upon fusion of an intermediate endocytic compartment (the multi-vesicular body (MVB)) with the plasma membrane or those released directly from the plasma membrane. Generally, the diameter of exosomes ranges between 30 to 150 nm. The term "exosomal" refers to compounds found on extracellular vesicles.

The present invention relates to the use of exosomal biomarkers and methods und kits using the same for diagnosis and prognosis of squamous cell carcinoma, and in particular head and neck squamous cell carcinoma (HNSCC).

According to the present invention, exosomal Pan-EV, Pan-CK, EpCAM, PD-L1 and CD45 are chosen as feasible biomarkers for the presence of squamous cell carcinoma or increased risk of development thereof in a subject. These biomarkers are therefore selected to provide the prognosis method according to the present invention for monitoring squamous cell carcinoma, especially in head and neck cancer patients.

The present invention therefore relates to an in vitro method for the diagnosis and/ or prognosis of squamous cell carcinoma or an elevated risk of future occurrence thereof, wherein the method uses detection of at least one exosomal biomarker correlating with a diagnosis of squamous cell carcinoma or an elevated risk of developing the same, the correlation taking into account the presence and level of the at least one biomarker in the sample as compared to a reference value.

In more detail, the method of the present invention comprises the following steps:
(i) obtaining a sample from a subject;
(ii) assaying the sample obtained in step (i) for the presence of at least one biomarker and determining a quantitative value of the least one selected biomarker in the test sample;
(iii) comparing the biomarker quantitative value obtained in step (ii) with the corresponding reference value, wherein the reference value is a quantitative value of the least one selected biomarker in a reference sample obtained from a healthy subject; and
(iv) evaluating the comparison results obtained in step (iii) for determining, whether a subject suffers from a squamous cell carcinoma or has an increased risk of developing the same.

According to the present invention, at least one biomarker being used as indicator for the occurrence of squamous cell carcinoma and risk analysis is an exosomal marker, which is selected from a group consisting of exosomal Pan-EV, Pan-CK, EpCAM, PD-L1 and CD45. In other words, the biomarker is a polypeptide or protein, which is present on the surface of exosomes, such as plasma exosomes. The presented biomarkers Pan-EV, Pan-CK, EpCAM, PD-L1 and CD45 are exosomal proteins and isoforms thereof.

According to an embodiment of the present invention, the sample is assayed for the presence and respective levels of more than one biomarker selected from the group consisting of exosomal Pan-EV, Pan-CK, EpCAM, PD-L1 and CD45.

In a preferred embodiment, the sample is assayed for the presence and respective levels of all selected biomarkers represented by exosomal Pan-EV, Pan-CK, EpCAM, PD-L1 and CD45.

In one embodiment, presence and/ or an increased level of exosomal Pan-EV, Pan-CK, EpCAM, PD-L1 and CD45 biomarkers in a test sample as well as their ratios may be used as indicator for a tumor stage of squamous cell carcinoma and for the assessment whether a subject has an increased risk of developing squamous cell carcinoma.

Preferably, the subject sample is tested for the levels of exosomal Pan-EV, Pan-CK, EpCAM, PD-L1 and CD45 and the values thereof are compared to those observed in a reference sample. Additionally, a percentage of Pan-EV+/CD45- or Pan-EV+/CD45+ with triple positive of PD-L1, Pan-CK and EpCAM biomarkers is determined for the subject sample and also compared to that of the reference sample. Elevated values for the exosomal Pan-EV, Pan-CK, EpCAM, PD-L1 and CD45 and an elevated percentage of triple positive Pan-EV+/CD45- or Pan-EV+/CD45+ in the tested sample relative to the reference sample correlate with and are therefore indicative for the presence of squamous cell carcinoma or an elevated risk of future occurrence thereof.

The other way round, this correlation also allows monitoring effectiveness of a tumor treatment of squamous cell carcinoma by detecting at least one of exosomal Pan-EV, Pan-CK, EpCAM, PD-L1 and CD45 in a sample obtained from a tumor patient, wherein decreased levels and or relative values (such as ratios, percentages) of the biomarkers, when compared to those values measured prior to treatment in the same patient, can be used as indicators that a chosen treatment route is successful.

In step (i) of the method of the present invention, a sample is obtained from a subject, which should be tested for the diagnosis and/ or prognosis of squamous cell carcinoma or an elevated risk of future occurrence thereof. Said sample, also called subject sample in this disclosure, may be any of saliva, blood, blood plasma, serum, urine or other body fluid. Most preferably, the sample is blood plasma.

As step (ii) is directed to the detection of presence and level of at least one exosomal biomarker in the sample, the method further includes a substep of enriching and/ or isolating exosomes from the sample prior to step (ii). Many methods for exosome isolation are known in the art. Preferably, the EXÖBead^{®} -based technique disclosed in document EP 3 602 055 A1 is applied for effectively isolating the exosomes with increased sensitivity of isolation, so that exosomes may be also effectively enriched in samples of a minimal volume, e.g., those of less than 10 ml.

It is well known that biomarkers are generally detectable by means of binding assays, which are biochemical assays providing a binding agent, such as an antibody, which can bind to the biomarker. Binding assays commonly use radioactive or fluorescent labeling for detecting binding of the biomarker to the agent. Binding assays may further use immobilization of the biomarker on solid assays having pre-printed antibody spots or may be done in solution.

Therefore, in step (ii) detecting the presence and level of at least one exosomal biomarker in the sample may be performed by using multiple antibodies analysis on the exosome-containing (enriched) sample. Preferably, plasma exosomes are used for the multiple antibodies analysis.

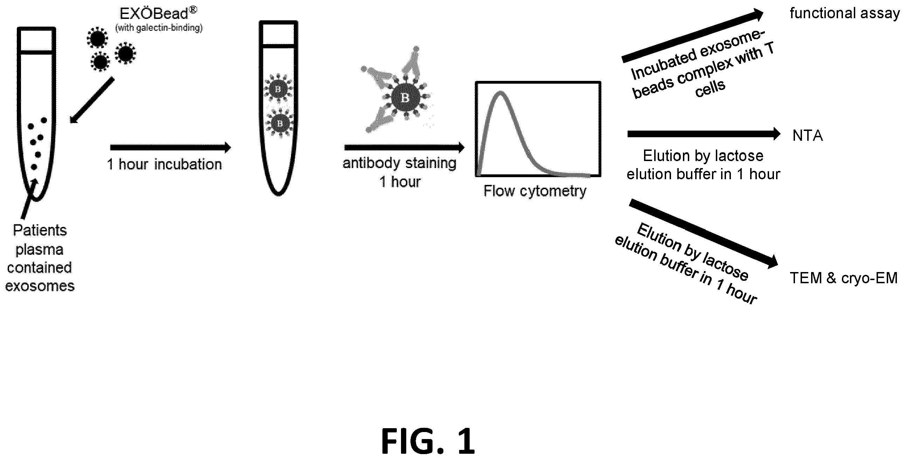

Multiple antibodies analysis may be implemented through corresponding exosome antibody arrays or using multiple antibody plotting, such as EXObead^{®}-based antibodies stained flow cytometry as used in a preferred embodiment of the present invention. Referring to FIG. 1, a workflow diagram is provided for further illustration, which generally shows procedure of such method for detecting biomarkers on exosomes by using EXÖBead^{®}. Accordingly, a patient sample, such as pre-processed blood, can be taken to isolate exosomes therefrom by adding EXÖBead^{®}. Further visualisation and experiments can be conducted by adding fluorescent antibodies. This process is defined in detail in document EP 3 602 055 A1 and therefore will not be repeated herein. Non-antibody coated EXÖBead^{®} may be provided, which, when used with an exosome-free buffer, enables efficient extraction of exosomes from the sample by forming exosome-bead complexes. By using an elution buffer that has a pH value in the range of the neutral and physiological pH (i.e., about 6.8 to 7.6), the integral exosomes may be released from the exosome-beads complexes for further analysis, such as functional assays.

The use of at least one exosomal biomarker according to the method of the present invention allows sensitive, specific and reliable detection and identification of head and neck squamous cell carcinoma (HNSCC).

According to certain examples of the present disclosure, the subject is a mammalian. Preferably, the sample is obtained from human.

In a preferred embodiment, the method of the present invention is therefore applied for head and neck cancer patients or patients suspected to have or being at risk of developing head and neck squamous cell carcinoma (HNSCC) to allow, e.g. early diagnosis, assessing tumor stage and/ or monitoring therapeutic effects.

In view of the foregoing, it has been found that a panel of up to five exosomal biomarkers is very useful for diagnosis and monitoring of squamous cell carcinoma, specifically HNSCC, in a subject sample obtained non-invasively. The present invention provides a method using at least one of these biomarkers.

As could be appreciated, a kit for in vitro diagnosis or prognosis of squamous cell carcinoma or an elevated risk of future occurrence thereof in a sample obtained from a subject also falls within the scope of the present disclosure.

According to the present disclosure, such screening kit comprises a reference sample for at least one biomarker selected from the group consisting of exosomal Pan-EV, Pan-CK, EpCAM, PD-L1 and CD45 and means for detecting presence and level of the at least one biomarker in a test sample.

According to certain embodiments, the kit comprises a reference sample covering all the exosomal biomarkers Pan-EV; Pan-CK; EpCAM; PD-L1 and CD45 and means for respectively detecting presence and levels of these biomarkers and, optionally, also means for determining a percentage of Pan-EV+/CD45- or Pan-EV+/CD45+ with triple positive of PD-L1, Pan-CK and EpCAM.

In one example, the kit may be an antibody array having pre-printed spots featuring respective antibodies for the selected exosome biomarkers. In the present case, this could be a 5 spot panel assay. The assay may further comprise additional control spots, e.g. for checking cellular contamination, as known in the art.

The kit according to the present invention may be further useful in an in vitro method for screening therapeutic antibodies for use in treating squamous cell carcinoma in a patient.

The therapeutic antibody screening method of the present disclosure is based on the observation that peripheral blood mononuclear cells (PBMCs) of a subject's sample show a specific response, when incubated with the subject's own exosomes or foreign exosomes, wherein said response is patient-specific, so that a sample obtained from a subject suffering from HNSCC may be screened for the most suitable therapeutic antibody for treating said subject by incubating a sample of the subject's PBMCs with the subject's own exosomes and potential pre-selected antibodies.

The therapeutic antibody screening method of the present disclosure comprises the following steps:
(i) providing a liquid biopsy sample from a subject, wherein the liquid biopsy sample is a whole blood sample;
(ii) incubating peripheral blood mononuclear cells (PBMCs) isolated from the sample provided in step (i) and analyzing presence of markers CTLA4 and CD69 before and after stimulation, said markers being indicators of T - cell activation;
(iii) respectively treating peripheral blood mononuclear cells (PBMCs) isolated from the sample provided in step (i) and / or the platelet-poor plasma of the liquid biopsy sample with an agent containing exosomes;
(iv) further treating the sample of step (iii) with an agent containing pre-selected antibodies;
(v) further analyzing the platelet-poor plasma of the liquid biopsy sample for the presence and amounts of exosomal markers Pan-EV, Pan-CK, EpCAM, PD-L1 and CD45; and
(iv) determining, on the basis of the results obtained in the foregoing steps, whether the conditions for therapeutic antibody positive response are met.

According to the present invention, the conditions for therapeutic antibody positive response are met, if the following four prerequisites are fulfilled: (1) the total exosome including tumor - derived exosome suppresses proliferation of the subject's own PBMCs, (2) a percentage of CTLA4- CD69+ is increased after stimulation, (3) a percentage of CTLA4+ CD69- is decreased after stimulation and (4) a percentage of CD45- EV with PanCK+, EpCAM+ and PD-L1 + for the patient's sample is higher than in a comparison sample obtained from healthy subjects.

According to certain embodiments, the exosomes in the agent of step (iii) may be exosomes obtained from the subject's sample. In another example, the exosomes in the agent of step (iii) may be exosomes obtained from a sample of another subject. The exosomes may be the total exosome including tumor-derived exosomes.

The pre - selected antibody in the agent of step (iv) may be any effective or potential therapeutic antibody available for the skilled person or may be a mixture of more than one therapeutically effective antibody.

According to one example of the present disclosure, the pre - selected antibody in the agent of step (iv) may be chosen from a group consisting of Durvalumab (PD-L1 inhibitor, Imfinzi^{®}), Atezolizumab (PD-L1 inhibitor, Tecentriq^{®}), Cetuximab (EGFR inhibitor, Erbitux^{®}), Nivolumab (PD1 inhibitor, Opdivo^{®}), Pembrolizumab (PD1 inhibitor, KEYTRUDA^{®}), Trastuzumab (HER2/neu innibitor, Herceptin^{®}) and these antibodies from other clones and / or mixtures thereof. However, as afore-mentioned, the therapeutic antibody screening method of the present invention shall not be limited to the exclusive use of one or more of these known therapeutic antibodies, but may be alternatively apply other antibodies depending on the desired applications.

The therapeutic antibody screening method of the present disclosure is advantageous, because it helps the treating physician to select the most promising antibody for therapy individually for each patient based on liquid biopsy. Moreover, selection of an effective therapeutic antibody according to the method of the present invention may be completed within 3 days. Such approach using liquid biopsy with tumor - derived exosome for fast therapeutic antibody screening has not been reported or studied so far.The following examples are provided to elucidate certain aspects of the present invention and to aid those of skilled in the art in practicing this invention. Without further elaboration, it is believed that one skilled in the art can, based on the description herein, utilize the present invention to its fullest extent.

### 1. MATERIALS AND METHODS

### 1.1. Sample collection

10 patients from the ENT clinic of the University hospital of Basel with HNSCC in early and advanced stages were included as well as a healthy control subjects. Blood was collected during operation and aftercare (EDTA, S-Monovette, Sarstedt, Germany). Isolation was conducted as described previously reported in the publication "Isolation of biologically-active exosomes from human plasma" by Muller, L.; Hong, C.S.; Stolz, D.B.; Watkins, S.C.; Whiteside, T.L. (J Immunol Methods 2014, 411, 55-65). Informed consent was obtained voluntarily from each patient enrolled in the study and ethics approval has been also obtained.

### 1.2 EXÖBead^{®} isolation

1 ml of pre-processed patient plasma (PPP) was directly diluted in 0.95 ml of 0.5 % exosome-free BSA in PBS (100,000 g, 4 °C, overnight centrifugation) and incubated with 50 µl of EXÖBead^{®} (9x108 1 µm particles/ml from Biovesicle Inc.) at 37 °C for 1 hour. Exosome-EXÖBead^{®} complexes were washed 2 times with 1 ml of 0.5 % exosome-free BSA in PBS by using magnets.

### 1.3 Exosomal surface marker staining

After removing the non-EVs fractions (EV: Extracellular Vesicles), exosome-EXÖBead^{®} complexes were resuspended in 100 µl of 1 ml 0.5% exosome-free BSA/ PBS and incubated with 100 µl of universal antibodies mixtures at 25 °C for 1 hour. The antibodies mixtures contained 3 µl of PE-PanEV antibodies (pre-mixed by PE-anti-CD63: H5C6 clone, PE-CD81: 5A6 clone and PE-CD9: HI9a clone), 4 µl of Alexa Fluor^{®} 488 anti-Pan Cytokeratin: C-11 (10 µg/ml), 5 µl of Brilliant Violet 421 ^{™} anti-human CD45 Antibody: HI30, 5 µl of APC anti-PD-L1 antibody: MIH1 and 5 µl of BV786 Mouse Anti-Human CD326 antibody: EBA-1. Antibodies stained exosomes-EXÖBead^{®} complexes were acquired on BD LSR Fortessa^{™} (BD Biosciences) and data were analyzed with FlowJo software (Tree Star, Ashland, OR).

### 1.4 Intracellular exosomal marker and non-EVs marker staining

Non-EVs fractions were collected after PPP incubating with EXÖBead^{®} and covalently-coupled to 1-Ethyl-3-[3-dimethylaminopropyl] carbodiimide hydrochloride (EDC) in 0.1 M 2-(N-Morpholino) ethanesulfonic acid (MES), pH 5.0, for 2 hours incubation at 25 °C. Non-EVs-bead complexes were further blocked by 0.5 % exosomes-free BSA in PBS (100,000 g, 4 °C for overnight centrifugation). Exosomes-EXÖBead^{®} complexes and the non-EVs-beads complex were further fixed by 200 µl of 4% paraformaldehyde in PBS for 10 minutes at 25 °C, penetrated by 200 µl of 0.1% Tween-20 in PBS for 10 minutes at 25 °C and blocked by 200 µl of 10% exosomes-free FBS in PBS (100,000 g, 4 °C for overnight centrifugation) for 30 minutes at 25 °C. Exosomes-EXÖBead^{®} complexes and non-EVs-beads complex were further resuspended in 100 µl of 1 ml 0.5% exosome-free BSA in PBS for antibodies staining and further incubated with 100 µl of universal antibodies mixtures at 25 °C for 1 hour. The antibodies mixtures contained 2.5 µg/ml of Alexa Fluor^{®} 488 anti-apolipoprotein A-I/ApoA1: 2083A and 5 µg/ml of PE anti-TSG101: EPR7130(B). Antibodies stained exosomes-EXÖBead^{®} complex/non-EVs-beads complex were acquired on BD LSRFortessa^{™} (BD Biosciences) and data were analyzed with FlowJo software (Tree Star, Ashland, OR).

### 1.5 Nanoparticle Tracking Analysis

EV numbers and sizes were determined by ZetaView^{®} Nanoparticle Tracking Analyzer PMX 110 (Particle Metrix GmbH, Germany). Briefly, EVs were diluted in PBS to a final volume of 1mL. For each measurement, two cycles were performed by scanning 11 positions each and capturing 60 frames per second under following settings: pre-acquisition parameters were set to a sensitivity of 80; shutter was set to 70; cell temperature was set to 25 °C and trace length to 15. After the capture, the videos were analyzed by the inbuilt ZetaView Software 8.05.11 SP1 with specific analysis parameters: minimum particle brightness: 20; Mminimum Size of 5 pixies, maximum size of 1000 pixies and PSD nm/Class of 10 and PSD Classes/Decade of 10.

### 1.6 Functional assay

CD4+-extraction was conducted from buffy coats received by Blutspendezentrum Basel as recommended by the manufacturer according to the manual with the Straight-FromTM Buffy Coat CD4 Micro Bead Kit (Product-No: 130-114-980 MACS Miltenyi Biotec, Auburn, USA). The T-cell activation was conducted according to the manual as decribed in the T Cell Activation/Expansion kit (Product-No: 130-091-441, MACS Miltenyi Biotec, Auburn, USA). 30 µl TEX were added to each well and gently mixed and further incubated with an antibody master mix containing 2.5 µl antibody/test of CD152 Monoclonal Antibody APC: 14D3, CD4 Monoclonal Antibody eFluor^{®} 450: SK3 and CD69 Monoclonal Antibody FITC: FN50 (Thermo Fisher Scientific, California, USA) for 50 min at 37°C. Analytical assay was performed with FlowCytometry (CytoFlex, Beckman Coulter, California, USA).

### 1.7 Pre - studies of therapeutic antibody screening

Around 50 mL of whole blood samples were obtained from patients with HNSCC (sample No. 59, 60, 61, 62, 63, 64, 65) and healthy control subjects (sample No. CL, C6, C7). Buffy coat was prepared from blood samples (sample No. 59, 60, 61) by centrifugation (at 800g) at room temperature for 10 min without brake. The plasma obtained during buffy coat preparation (around 10 mL) was saved at -80 °C. 10 mL of buffy coat was mixed with 20 mL PBS, then 10 mL of Ficoll were added into 30 ml of PBS-buffy coat to isolate PBMCs by centrifugation (at 400g) for 40 minutes at room temperature without brake. Then taking the PBMCs layer (around 5-10 mL) and washing the same with PBS once (at 350g, 10 minutes at room temperature) and resuspending the pellets in 5 ml of 0.22 µm filtered ACK RBCs lysis buffer at room temperature for 3 minutes of incubation time. Afterwards, at least 25 mL of completed medium (RPMI 1640 with 10% FBS/1% P/S) were immediately added to neutralize ACK buffer and centrifugated (at 350g) for 10 minutes at room temperature. The sample was washed again by resuspending pellets in 10 ml completed medium. After resuspending cells in 20 mL completed medium and overnight culturing, the isolated cells (PBMCs) were counted and seeded in the concentration of 2x10^{∧}6 cells/ml (total 0.5 ml of completed medium in 48 wells). At the meantime, 1 mL of platelet poor plasma (collected after centrifugation at 10000 g for 30 minutes at 4°C) was mixed with EXObead at room temperature for 1 hour, followed by washing the exosome-beads complex and eluting exosomes from the exosome-beads complex by adding 200 µl 0.3M lactose in PBS elution buffer at room temperature for 1 hour. Wells with seeded PBMCs were further treated with / without 25 µL eluted exosome, with / without 1 µg/ml of antibody and 5 ng/ml of PMA/ 1µM of lonomycin in the total 1 ml of completed medium. After stimulation/treatment, PBMC cell pellets were collected and stained with 100 µl Zombie NIR^{™} Viability Dye (Biolegend) in the 1:1000 dilution at room temperature for 15 minutes. Viability Dye stained cells were washed two times with 1% BSA in PBS. Viability Dye stained cells were further stained with an antibody master mix containing 2.5 µl antibody/test of PE anti-human CD3 Antibody (Clone: UCHT1, Biolegend), CD152 Monoclonal Antibody APC (Clone: 14D3, Thermo Fisher Scientific), CD4 Monoclonal Antibody eFluor^{®} 450 (Clone: SK3, Thermo Fisher Scientific) and CD69 Monoclonal Antibody FITC: FN50 (Thermo Fisher Scientific, California, USA) for 60 min at 4°C. Analytical assay was performed with Flow Cytometry (CytoFlex, Beckman Coulter, California, USA). Another 0.5 ml platelet poor plasma (collected after 10000 g at 30 minutes 4 °C) was used to perform 5 color staining in another day to determine MFI of PanEV, CD45, PanCK, EpCAM and PD-L1. Flow cytometry data were also evaluated to determine percentage of CD45- EV with PanCK+, EpCAM+ and PD-L1 +.

### 1.8 Statistical analysis

Data were analyzed and statistically evaluated with Prism version 8 (GraphPad, California, USA). Further analysis was conducted by FlowJo software (Tree Star, Ashland, OR).

### 2. RESULTS

### 2.1 Exosome isolation by EXÖBead^{®}

Again referring to the scheme shown in FIG. 1, for exosome isolation from patient plasma, EXÖBead^{®} were mixed with patient plasma after differential centrifugation up to 10000 g. For the experiments 1 ml of plasma was collected. Binding of EXÖBead^{®} to exosomes in plasma is strong why only 1 hour of incubation time was needed. Further antibody staining needed 1 hour. After capturing of exosomes on beads multiple experimental studies were conducted. Through the bigger size of the beads (1µm, Chemicell, Berlin, Germany and Ocean NanoTech, California, USA) exosomes were indirectly visualizable by flow cytometry. Binding between exosomes and EXÖBead^{®} was dissolved by adding lactose elution buffer for another hour to conduct further studies like transmission electronic microscopy (TEM).

### 2.2 Quality of exosomes recovered

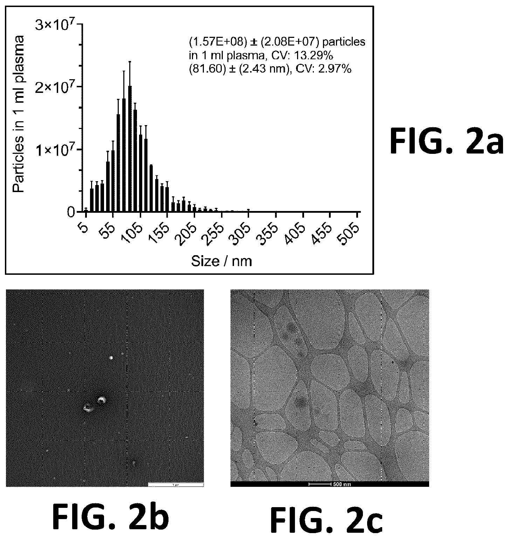

Exosomes isolated by EXÖBead^{®} were evaluated with flow cytometry, nano tracking assay and TEM. Some of the results are exemplarily depicted in FIG. 2a, which is a diagram representing triplicate results of measured particles in plasma by ZetaViewer from one donor, FIG. 2b showing eluted exosome with EXÖBead^{®} in transmission electron microscopy (TEM) and FIG. 2c showing eluted exosome with EXÖBead^{®} in cryogenic electron microscopy (cryo-EM). Isolated exosomes dissolved from EXÖBead^{®} appear as cup-shaped, membrane bound vesicles ranging from 20-100 nm. Exosomes showed to be intact in TEM and cryo-EM. Nano tracking analysis (ZetaViewer) provided comparable and reproducible results represented by triplicate measures from one donor. The majority of measured particles were small EV like vesicles ranging from 30-200nm. Some contamination was seen with non-EV like structures or large EV like vesicle (200-1000nm). Exosomes after recovery were quantified in µg protein normalized to 1 ml of plasma.

### 2.3 Exosomal surface markers

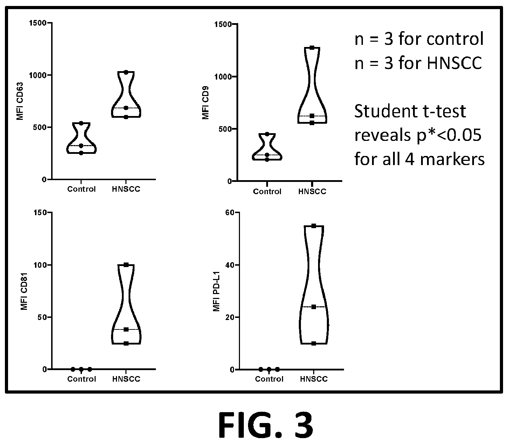

Recovered exosomes were controlled for typical exosomal surface markers by flow cytometry. Flow cytometry discriminated single beads from aggregated beads and was able to quantify exosomal protein levels. Reference is made to FIG. 3, which is a schematic diagram showing MFI (mean fluorescence intensity) of exosome-EXÖBead^{®} complex from healthy donors and patients. According to the results, typical exosomal surface markers, such as CD63, CD9 and CD81 were expressed on recovered EXÖBead^{®} isolated exosomes and were significantly increased in HNSCC patients compared to healthy donors (p<0.05) for all surface markers. Also PD-L1, an important diagnostic surface marker, was significantly increased in tumor patients compared to the control group.

### 2.4 Intracellular exosomal markers

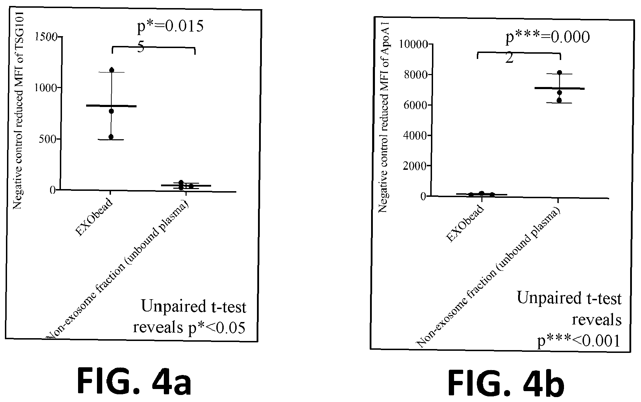

Further reference is made to FIG. 4a and 4b. Besides extracellular markers also TSG101 as another typical intravesicular exosomal marker was tested. FIG. 4a is a schematic diagram showing control of exosomal intravesicular marker TSG101. In summary, experiments were feasible and TSG101 was expressed in samples from HNSCC patients after exosome isolation and recovery by EXÖBead^{®}. Levels of isolates were significantly increased compared to the levels in unbound patients' plasma.

Another problem very often faced is the complexity of blood samples and thus contamination. ApolipoproteinA1, being a marker for contamination of exosomal preparations, therefore was tested as well. FIG. 4b is a schematic diagram showing control of non-EVs marker ApoA1. It can be seen that ApoA1 level was high in residual plasma compared to almost none in the EXÖBead^{®} fraction.

### 2.5 HNSCC specific exosomal markers

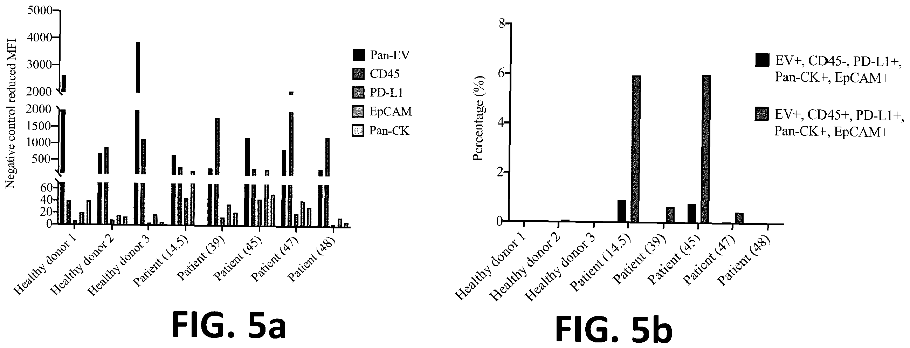

A panel for various exosomal markers was set up: Pan-EV, PD-L1, EpCAM, Pan-CK and CD45. These were tested after rapid exosomal isolation by EXÖBead^{®}. FIG. 5a is a schematic diagram showing MFI of exosome-EXÖBead^{®} complex from healthy donors and patients. Figure 5b is a schematic diagram showing percentage of EV+/ CD45- or EV+/CD45+ with triple positive HNSCC biomarkers, PD-L1, Pan-CK and EpCAM. PD-L1 in control was low compared to HNSCC exosomes. Similar results were shown with exosomal marker EpCAM being increased in cancer exosomes. Pan-CK and CD45 was present in control as well as tumor exosomes and therefore alone is not tumor-specific.

### 2.6 Functions of plasma-derived exosomes

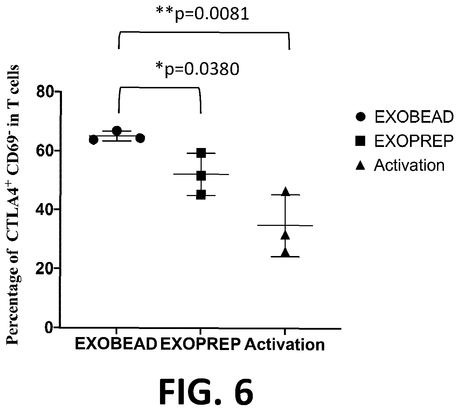

To evaluate the biological activity and functional competence of the isolated and recovered exosomes by EXÖBead^{®} as well as conventional assay (SEC and UC) the tumor-derived exosomes (TEX) were co-incubated with activated T-cells (CD4+-cells) to measure immunosuppression. Percentage of CTLA4+ CD69- T-responder cells was measured by flow cytometry after co-incubation. The results are shown in FIG. 6. T-cells were activated by TEX isolated by EXÖBead^{®} as well as conventional methods. However only isolated and recovered exosomes by EXÖBead^{®} compared to control showed significant change.

To sum up, compared with the existing exosome isolation kit on the market, eluted exosome by EXÖBead^{®} can keep their activity.

Moreover, since cancer patients show a significant higher level of exosomes compared to healthy controls, active disease and therefore higher tumor load can be associated with higher exosomal load. After completion of therapy, a level of exosomes decreased and therefore shows a disease dependent behaviour. The present invention using a panel of various exosomal markers selected from Pan-EV, PD-L1, Ep-CAM, Pan-CK and CD45 provides a poweful and efficient tool in tumor diagnosis and therapy tracking of HNSCC. Preferably, isolated exosomes for biomarker analysis are obtained by means of EXÖBead^{®} isolation, since this technique provides functional-competent exosomes representing the intracellular exosomal markers, having low non-exosome contamination and the sample comprising high reproducible exosome-like particles.

In conclusion, the present method and the kit provide an easy, fast and reproducible method for diagnosis and monitoring of cancer patients with focus on HNSCC.

### 2.7 Therapeutic antibody screening

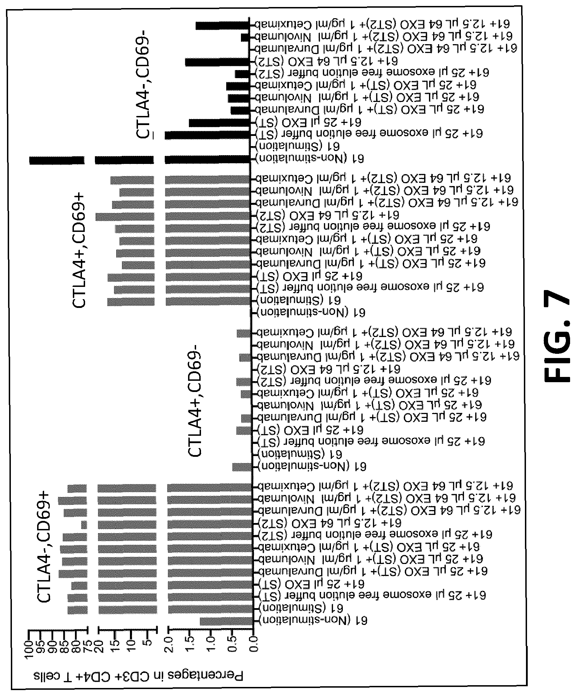

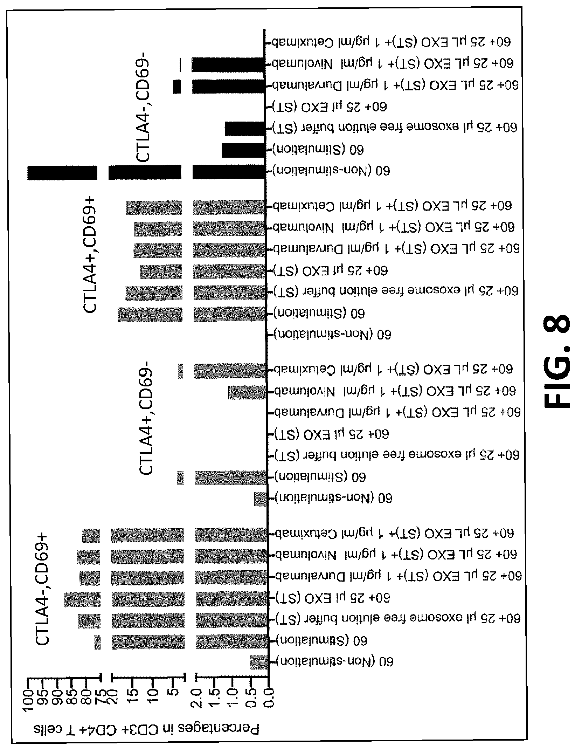

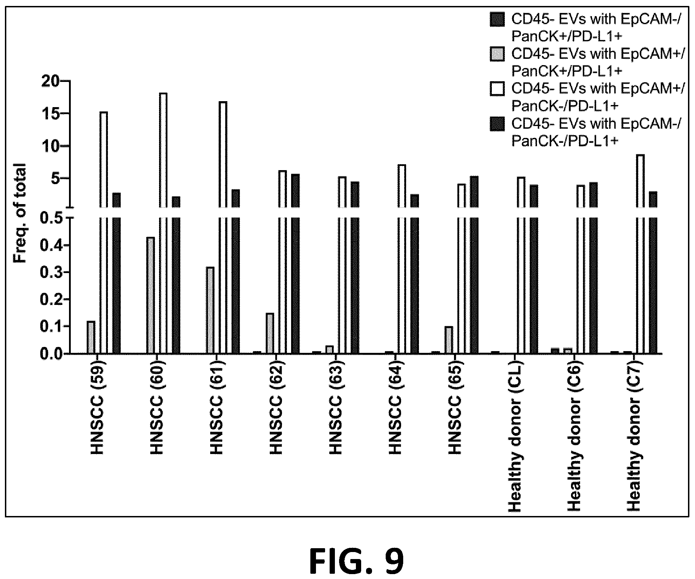

In FIG. 8 and 9, the term "ST" defines those samples with PBMCs stimulation with PMA and ionomycin. Referring to FIG. 7 to 9, it has been found that for therapeutic antibody positive response the following four prerequisites should be fulfilled: (1) the total exosome including tumor - derived exosome suppresses proliferation of the subject's own PBMCs, (2) a percentage of CTLA4- CD69+ is increased after stimulation, (3) a percentage of CTLA4+ CD69- is decreased after stimulation and (4) a percentage of CD45- EV with PanCK+, EpCAM+ and PD-L1 + for the patient's sample is higher than in a comparison sample obtained from healthy subjects.

The present method thus can provide an easy, fast and reproducible method for screening and selecting the most effective antibody / antibodies for individual therapy of a patient suffering from HNSCC.

## Claims

1. An in vitro method for the diagnosis and / or prognosis of squamous cell carcinoma or an elevated risk of future occurrence thereof, said method comprising detection of at least one biomarker correlating with the occurrence of squamous cell carcinoma or an elevated risk of developing the same, the correlation taking into account the presence and level of the at least one biomarker in the sample as compared to a reference value, wherein said method comprises the following steps:
(i) providing a sample from a subject;
(ii) assaying the sample provided in step (i) for the presence of at least one biomarker and determining a quantitative value of the least one selected biomarker in the sample;
(iii) comparing the quantitative biomarker value obtained in step (ii) with the corresponding reference value, wherein the reference value is a quantitative value of the least one selected biomarker in a reference sample obtained from a healthy subject; and
(iv) determining, on the basis of the comparison results obtained in step (iii), whether the subject is likely to have squamous cell carcinoma or has an elevated risk of developing the same,
**characterized in that**
the at least one biomarker is an exosomal marker, which is selected from a group consisting of exosomal Pan-EV; Pan-CK; EpCAM; PD-L1 and CD45.

2. The in vitro method according to claim 1, wherein Pan-EV; Pan-CK; EpCAM; PD-L1 and CD45 are exosomal proteins and isoforms thereof.

3. The in vitro method according to any of the preceding claims, wherein the sample is assayed for the presence and level of multiple biomarkers.

4. The in vitro method according to any of the preceding claims, wherein exosomal Pan-EV; Pan-CK; EpCAM; PD-L1 and CD45 are detected in the sample.

5. The in vitro method according to any of the preceding claims, wherein presence or increased level of exosomal Pan-EV; Pan-CK; EpCAM; PD-L1, CD45 and their ratio are indicative of a tumor stage.

6. The in vitro method according to any of the preceding claims, comprising assaying the subject sample for presence and levels of exosomal Pan-EV; Pan-CK; EpCAM; PD-L1 and CD45 in step (ii) and comparing the values of exosomal Pan-EV; Pan-CK; EpCAM; PD-L1 and CD45 and a percentage of Pan-EV+/CD45- or Pan-EV+/CD45+ with triple positive of PD-L1, Pan-CK and Ep-CAM biomarkers in the subject sample with those from the reference sample in step (iii), wherein elevated values for the exosomal Pan-EV; Pan-CK; EpCAM; PD-L1 and CD45 and percentage of triple positive Pan-EV+/CD45- or Pan-EV+/CD45+ in the tested sample relative to the reference sample correlate with presence of squamous cell carcinoma or an elevated risk of future occurrence thereof.

7. The in vitro method according to any of the preceding claims, wherein the subject sample obtained in step (i) is selected from a group consisting of saliva, blood, blood plasma, serum and urine.

8. The in vitro method according to claim 7, wherein the subject sample is blood plasma.

9. The in vitro method according to any of the preceding claims, wherein step (ii) is performed by using multiple antibody plotting.

10. The in vitro method according to any of the preceding claims, wherein EXObead^{®}-based antibodies stained flow cytometry is used to detect the at least one exosomal biomarker in the sample.

11. The in vitro method according to any of the preceding claims, wherein the sample obtained in step (i) is from a head and neck cancer patient or subject suspected to have or being at risk of developing head and neck squamous cell carcinoma (HNSCC).

12. A kit for diagnosis and / or prognosis of squamous cell carcinoma or an elevated risk of future occurrence thereof, especially for carrying out the method of any of the preceding claims, comprising a reference sample for at least one biomarker selected from a group consisting of exosomal Pan-EV; Pan-CK; EpCAM; PD-L1 and CD45 and means for detecting presence and level of the at least one biomarker.

13. A kit according to claim 12, comprising a reference sample for all the exosomal biomarkers Pan-EV; Pan-CK; EpCAM; PD-L1 and CD45 and means for detecting these biomarkers and for determining a percentage of Pan-EV+/CD45- or Pan-EV+/CD45+ with triple positive of PD-L1, Pan-CK and EpCAM.

14. An in vitro method for screening therapeutic antibodies for use in treating squamous cell carcinoma, said method comprising the following steps:
(i) providing a liquid biopsy sample from a subject, wherein the liquid biopsy sample is a whole blood sample;
(ii) incubating peripheral blood mononuclear cells (PBMCs) isolated from the sample provided in step (i) and analyzing presence of markers CTLA4 and CD69 before and after stimulation;
(iii) respectively treating peripheral blood mononuclear cells (PBMCs) isolated from the sample provided in step (i) and / or the platelet - poor plasma of the liquid biopsy sample with an agent containing exosomes;
(iv) further treating the sample of step (iii) with an agent containing pre - selected antibodies;
(v) analyzing the platelet - poor plasma of the liquid biopsy sample for the presence and amounts of exosomal markers Pan-EV, Pan-CK, EpCAM, PD-L1 and CD45;
(iv) determining, on the basis of the results obtained in the foregoing steps, whether the conditions for therapeutic antibody positive response are met.

15. The in vitro method according to claim 14, wherein the conditions for therapeutic antibody positive response are met, if the total exosome including tumor - derived exosome suppresses proliferation of the subject's own PBMCs, a percentage of CTLA4- CD69+ being increased after stimulation and a percentage of CTLA4+ CD69- being decreased after stimulation and a percentage of CD45- EV with PanCK+, EpCAM+ and PD-L1 + being higher than in healthy subjects.

16. The in vitro method according to claim 14, wherein the exosomes in the agent of step (iii) are exosomes obtained from the subject's sample or are exosomes obtained from a sample of another subject, the exosomes being the total exosome including tumor - derived exosomes.

17. The in vitro method according to any of claims 14 to 16, wherein the pre - selected antibody in the agent of step (iv) is selected from a group consisting of Durvalumab (PD-L1 inhibitor, Imfinzi^{®}), Atezolizumab (PD-L1 inhibitor, Tecentriq^{®}), Cetuximab (EGFR inhibitor, Erbitux^{®}), Nivolumab (PD1 inhibitor, Opdivo^{®}), Pembrolizumab (PD1 inhibitor, KEYTRUDA^{®}) and Trastuzumab (HER2/neu inhibitor, Herceptin^{®}) and these four antibodies from other clones.
